# EUROPÄISCHE PATENTANMELDUNG

(11) **EP 2 626 089 A1**
(43) Veröffentlichungstag der Anmeldung: **14.08.2013**
(21) Anmeldenummer: 12000865.1
(22) Anmeldetag: 10.02.2012
(51) Int. Cl.: A61L 26/00

(54) **Flüssige Zubereitung zur Abdeckung oder Behandlung von Hautpartien**

(71) Anmelder: LTS LOHMANN Therapie-Systeme AG, 56626 Andernach (DE)
(72) Erfinder: Schnitzler, Iris, D-53127 Bonn (DE); Klein, Christina, D-56727 Mayen (DE)
(74) Vertreter: Schweitzer, Klaus

(57) **Zusammenfassung**

Beschrieben ist eine flüssige Zubereitung zur Abdeckung oder Behandlung von Hautpartien, die nach dem Auftragen auf die Haut einen transparenten oder durchscheinenden, zusammenhängenden Film ergibt. Sie enthält mindestens eine filmbildende Nitrocellulose mit einem Stickstoffgehalt von 8,0 bis 12,5 Gew.-%. Der Anteil an Nitrocellulose beträgt allgemein 10 bis 30 Gew.-%, bezogen auf das Gesamtgewicht der Zubereitung. Sie kann darüber hinaus mindestens einen pharmazeutischen oder kosmetischen Wirkstoff, vorzugsweise ein Antiseptikum enthalten, insbesondere Chlorhexidin, Chlorhexidindigluconat, Chlorhexidindiacetat, Benzalkoniumchlorid, Polyvinylpryrrolidon-lod (PVP-Iod), Polyhexanid und/oder Octenidindihydrochlorid. Beschrieben ist auch ein Stift (Applikator), mit dem sich die Zubereitung auf die Haut aufbringen lässt.

## Beschreibung

Die Erfindung betrifft eine flüssige Zubereitung, die nach dem Aufbringen auf die Haut einen wasserfesten Film ergibt und zur Behandlung von Hautwunden, Abschürfungen usw. geeignet ist. Sie betrifft daneben eine Vorrichtung (Applikator) zum Aufbringen der Zubereitung auf die Haut.

Neben den üblichen Pflastern sind Sprühpflaster und flüssige Zubereitungen bekannt. Eine solches Sprühpflaster ist beispielsweise in der DE 10 2008 048 338 A1 und DE 10 2008 060 904 A1 offenbart. Es enthält Silikon-Acrylat-Polymere, Silikonether und ein gasförmiges oder flüssiges Trägermittel. Das Trägermittel ist ein Alkan oder Alken mit 3 bis 20 Kohlenstoff-Atomen und oder ein Ether. Als Nachteil der Flüssig-Pflaster gegenüber den Sprühpflastern ist ein unzureichender Verlauf und die Bildung von zu dünnen Filmen auf der Haut aufgrund einer zu geringen maximalen Polymerkonzentration angegeben.

Gegenstand der WO 2006/010222 A1 ist eine Zusammensetzung in Form einer Paste oder Creme, eines Gels, einer Flüssigkeit oder eines Aerosols, die einen wasserfesten, flexiblen Film auf der Haut ergibt. Wesentliche Bestandteile der Zusammensetzung sind ein natürlicher Gummi, insbesondere Schellack, und ein cellulosischer Verdicker. Konkret erwähnt als Verdicker ist Ethylcellulose. Die Zusammensetzung kann darüber hinaus Pigmente und/oder therapeutisch wirksame Mittel zur Behandlung von Hautkrankheiten enthalten.

In der JP 2010-235471 A ist eine Zusammensetzung offenbart, die auf die Haut aufgebracht wird und dort einen transparenten oder durchscheinenden Film ergibt. Die Zusammensetzung enthält etwa 5 bis 10 Gew.-% an Nitrocellulose, daneben Kolophonium und ein wasserlösliches Polymer, beispielsweise Hydroxypropylcellulose, Polyacrylsäure oder Polyvinylalkohol. Als Lösungsmittel enthält die Zusammensetzung Isopentylacetat, Isobutylacetat und/oder Aceton. In einem Vergleichsbeispiel dieser JP-A wird eine Zusammensetzung beschrieben, die 7 Gew.-% Nitrocellulose ohne weitere polymere Zusatzstoffe enthält.

Es bestand daher die Aufgabe, ein Flüssigpflaster bereitzustellen, das die beschriebenen Nachteile nicht mehr aufweist. Es soll einfach aufzutragen sein, insbesondere mit einem Roller, schnell trocknen und hinreichend dicke, wasserfeste, flexible Filme auf der Haut ergeben. Die Filme sollen dabei ausreichend durchlässig für Sauerstoff und insbesondere Wasserdampf sein. Beim Auftragen soll das unangenehm brennende Gefühl auf der Haut bzw. Wunde, das bei Applikation von Sprühpflastern auftritt, vermieden oder zumindest auf ein erträgliches Maß reduziert werden. Die Filme sollen zudem ausreichend fest an der Haut haften, und sie sollen sich möglichst in einem Stück und ohne Anwendung von Lösemitteln wieder abziehen lassen.

Gelöst werden konnte die Aufgabe überraschenderweise durch die Verwendung von Nitrocellulose als film bildendem Bestandteil der flüssigen Zusammensetzung. Gegenüber (Meth)acrylat-Polymeren zeigen Nitrocellulosen wesentlich bessere Eigenschaften, insbesondere ergeben letztere Filme mit einem wesentlich besseren Zusammenhalt, so dass sie sich in einem Stück wieder abziehen fassen. Sie lassen sich zudem kombinieren mit Wirkstoffen, die die Wundheilung fördern.

Gegenstand der Erfindung ist demgemäß eine flüssige Zusammensetzung, die als filmbildende Komponente mindestens ein Cellulosederivat enthält und die nach dem Auftragen auf die Haut einen transparenten oder transluzenten, zusammenhängenden Film ergibt, die dadurch gekennzeichnet ist, dass Nitrocellulose das alleinige Celullosederivat ist und der Anteil an Nitrocellulose(n) 10 bis 30 Gew.-%, bezogen auf das Gesamtgewicht der Zusammensetzung, beträgt.

Der Anteil an Nitrocellulose(n) in der erfindungsgemäßen flüssigen Zusammensetzung beträgt vorzugsweise 12 bis 28 Gew.-%, insbesondere 15 bis 23 Gew.-%, jeweils bezogen auf das Gesamtgewicht der flüssigen Zusammensetzung.

Die Nitrocellulose weist allgemein einen Stickstoffgehalt von weniger als 12,6 Gew.-% auf, sie fallen also nicht unter die Bestimmungen des deutschen Sprengstoff-Gesetzes, und sie müssen auch nicht phlegmatisiert werden. Der Stickstoffgehalt beträgt bevorzugt etwa 8 bis 12,5 Gew.-%, besonders bevorzugt etwa 10,0 bis 12,4 Gew.-%, speziell 11,0 bis 12,3 Gew.-%. Nitrocellulosen mit einem solchen Stickstoff-Anteil sind kommerziell erhältlich, beispielsweise von Dow Wolff Cellulosics GmbH.

Überraschenderweise zeigen Nitrocellulosen auch gegenüber anderen CelluloseDerivaten, speziell Celluloseethern wie Methylcellulose, in Flüssig-Pflastern verbesserte Eigenschaften, wie die nachfolgenden Beispiele und Vergleichsbeispiele zeigen.

Die erfindungsgemäße Zusammensetzung enthält keine wasserlöslichen Polymere, wie sie in der eingangs genannten JP 2010-235471 A beschrieben sind.

In einer bevorzugten Ausführungsform enthält die flüssige Zusammensetzung (C1 - C10)-Alkylester mehrwertiger Carbonsäuren, insbesondere mehrwertiger Hydroxycarbonsäuren, insbesondere Triethylcitrat. Diese Ester verbessern die Flexibilität der Nitrocellulosefilme deutlich.

Als Lösungsmittel für die erfindungsgemäße flüssige Zusammensetzung sind Ethanol, Ethylacetat, Propanol, Isopropanol, 2-Phenoxy-ethanol, Aceton, Butylacetat und Mischungen davon geeignet. In Wasser sind Nitrocellulosen praktisch unlöslich. Wasser kann jedoch in untergeordneten Mengen (bis 15 Gew.-%, bezogen auf das Gesamtgewicht der Lösungsmittel) in der flüssigen Zusammensetzung enthalten sein.

In bevorzugten Ausführungsformen enthält die flüssige Zusammensetzung als weiteren Bestandteil mindestens einen pharmazeutischen oder kosmetischen Wirkstoff, vorszugsweise ein Antiseptikum wie Chlorhexidin, Chlorhexidindigluconat, Chlorhexidindiacetat, Benzalkoniumchlorid, Polyvinylpryrrolidon-lod (PVP-Iod), Polyhexanid oder Octenidindihydrochlorid. Diese Wirkstoffe sind wasserlöslich. Ein Anteil an Wasser in der flüssigen Zusammensetzung ist dann besonders vorteilhaft. Je nach Art des wasserlöslichen Wirkstoffs bzw. der Kombination wasserlöslicher Wirkstoffe, beträgt der Anteil an Wasser an dem Gesamtgewicht der Lösungsmittel bis zu 15 Gew.-%, bevorzugt 5 bis 10 Gew.-%.

In einer weiteren Ausführungsform enthält die Zusammensetzung Pigmente und/oder Farbstoffe, mit denen sich Hautverfärbungen und kleinere Wunden verdecken lassen. Geeignete, pharmakologisch unbedenkliche Farbstoffe bzw. Pigmente sind beispielsweise Titandioxid (E 171), Pflanzenkohle (E 153), Grün S (E 142), Lycopin (E 160d), Lutein (E 161b), Betanin (E 162), Anthocyane (E 163), Eisenoxide (E 172).

Die erfindungsgemäße Zusammensetzung ist bevorzugt eine echte physikalische Lösung, gegebenenfalls kann sie, in untergeordneten Anteilen, dispergierte und/oder suspendierte Bestandteile enthalten.

Die erfindungsgemäße flüssige Zusammensetzung weist zweckmäßig eine Viskosität im Bereich von 1000 bis 40000 mPa·s, bevorzugt 5000 bis 15000 mPa·s, auf (bestimmt mittels Rheometer HAAKE RheoWin 4.30.0001), damit sie ausreichend fließfähig, jedoch nicht zu dünnflüssig ist. Eine solche Viskosität ist zweckmäßig, damit eine ausreichende Menge auf die zu behandelnde Hautpartie aufgebracht werden kann, ohne zu stark zu verlaufen. Nach dem Trocknen ergibt die flüssige Zusammensetzung auf der Haut einen festen, flexiblen, nicht-klebrigen, in der Regel zudem transparenten Film. Nach dem Trocknen hat der Film zweckmäßig eine Dicke im Bereich von etwa 10 bis 100 µm, entsprechend der aufgetragenen Menge der flüssigen Zusammensetzung.

Der getrocknete Film ist durchlässig für Sauerstoff und insbesondere Wasserdampf. Die Wasserdampfdurchlässigkeit (water vapor transmission rate, WVTR) wurde bestimmt nach EN 13726-2 (20 ml Wasser, Probengröße 20 cm²) und beträgt allgemein 400 bis 1350 g/m²/24h.

Gegenstand der vorliegenden Erfindung ist daneben eine Vorrichtung (Applikator) zur Verpackung und zum Aufbringen einer flüssigen Zusammensetzung, die eine vorgegebene Menge der beschriebenen flüssigen Zusammensetzung enthält und diese dann kontrolliert abgeben kann. Sie weist einen die Zusammensetzung enthaltenden Behälter auf und ist mit einer Auftragskonstruktion versehen. Der Applikator ist vorzugsweise ein Stift (Pen) mit einem mechanisch betätigten Kolben und einer elastischen, mit mindestens einer Austrittsöffnung, vorzugsweise mit mehreren Austrittsöffnungen, versehenen Spitze. Durch den von dem Kolben erzeugten Druck wird die flüssige Zusammensetzung aus der Spitze herausgedrückt. Die Spitze ist in einer besonderen Ausgestaltung der Erfindung als ein gelöcherter Gummistopfen ausgebildet. Die Spitze kann auch mit einer beweglich gelagerten Kugel versehen sein, die die Flüssigkeit verteilt, wie bei den bekannten "Roller"-Stiften. Der Applikator kann mit einem Deckel versehen sein, der die Auftragskonstruktion verschließen kann.

Mit einem solchen Applikator kann die erfindungsgemäße Zusammensetzung auch in der Nähe der Augen aufgebracht werden, wo Sprühpflaster nicht einsetzbar sind.

Die Erfindung betrifft weiterhin ein Verfahren zum Auftragen der erfindungsgemäßen Zusammensetzung auf die Haut und die Verwendung der vorerwähnten Vorrichtung zur Verpackung und zum Auftragen der erfindungsgemäßen Zusammensetzung.

Das nachfolgende Beispiel dient zur Illustration der Erfindung, ohne dass diese darauf beschränkt wäre. Prozente sind darin als Gewichtsprozente zu verstehen, soweit nicht anders angegeben oder aus dem Zusammenhang unmittelbar ersichtlich.

### Beispiel 1

4,5 g Nitrocellulose (Nitrocellulose E 950 von Dow Wolff Cellulosics GmbH) wurden in einer Mischung aus 10 g Ethylacetat und 7 g Ethanol unter Rühren gelöst. Anschließend wurden 3 g Triethylcitrat und dann 0,75 g PVP-Iod zu der Lösung hinzugegeben und homogenisiert. Es bildete sich ein homogenes Gel.

Das Gel ließ sich auf der Haut gut verteilen, die dabei gebildete Gelschicht trocknete gleichmäßig und schnell. Der resultierende Film war weich und flexibel. Das spätere Ablösen des Films gestaltete sich einfach. Er ließ sich in einem Stück abziehen.

### Beispiel 2 (Vergleich)

4,25 g eines kationischen Copolymers auf Basis von Dimethylaminoethylmethacrylat, Butylmethylacrylat und Methylmethacrylat (®Eudragit E PO) wurden in 4,25 g Ethanol gelöst. Anschließend wurde 1 g einer 20 %-igen wässrigen Chlorhexidin-digluconat-Lösung hinzugegeben und die Mischung homogenisiert. Anschließend wurde 0,5 g Triethylcitrat hinzugegeben und ebenfalls homogenisiert. Es entstand ein homogenes Gel, das optisch sehr ansprechend war. Das Gel wies zudem einen leicht unangenehmen Geruch auf.

Das Gel ließ sich auf der Haut problemlos verteilen. Die Gel-Schicht trocknete zügig. Während des Trocknens war die Oberfläche des Films zunächst leicht klebrig, der vollständig getrocknete Film war jedoch nicht mehr klebrig. Der anfangs flexible Film versprödete nach dem Trocknen.

Der Film ließ sich nur schwer von der Haut entfernen. Er ließ sich nicht in einem Stück abziehen, er musste vielmehr unter Verwendung von Ethanol mühsam entfernt werden.

### Beispiel 3 (Vergleich)

13,5 g einer 12,5 %-igen Lösung von Methylcellulose (®Metolose 60SH60 von ShinEtsu) in Ethanol/Wasser (1:1, v/v) wurden mit 7,5 g Ethanol verrührt. Dann wurden 2,5 g einer 20 %-igen wässrigen Chlorhexidin-digluconat-Lösung und 1,5 g Triethylcitrat hinzugefügt und die Mischung homogenisiert. Es bildete sich ein homogenes, farblos transparentes Gel.

Das Gel ließ sich auf der Haut sehr gut verteilen, das Trocknungsverhalten war zufriedenstellend. Der getrocknete Film haftete gut an der Haut, war jedoch etwas zu hart. Er konnte nicht in einem Stück von der Haut abgezogen werden, die Rückstände konnten jedoch leicht abgespült werden.

## Patentansprüche

1. Flüssige Zusammensetzung, die als filmbildende Komponente mindestens ein Cellulosederivat enthält und die nach dem Auftragen auf die Haut einen transparenten oder transluzenten, zusammenhängenden Film ergibt, **dadurch gekennzeichnet, dass** Nitrocellulose das alleinige Celullosederivat ist und der Anteil an Nitrocellulose(n) 10 bis 30 Gew.-% bezogen auf das Gesamtgewicht der Zusammensetzung beträgt.

2. Zusammensetzung gemäß Anspruch 1, **dadurch gekennzeichnet, dass** der Anteil an Nitrocellulose(n) 12 bis 28 Gew.-%, vorzugsweise 15 bis 23 Gew.-%, beträgt, jeweils bezogen auf das Gesamtgewicht der flüssigen Zusammensetzung.

3. Zusammensetzung gemäß Anspruch 1 oder 2, **dadurch gekennzeichnet, dass** die Nitrocellulose einen Stickstoffgehalt von weniger als 12,6 Gew.-%, bevorzugt 8 bis 12,5 Gew.-%, besonders bevorzugt etwa 10,0 bis 12,4 Gew.-%, speziell 11,0 bis 12,3 Gew.-%, aufweist.

4. Zusammensetzung gemäß einem oder mehreren der Ansprüche 1 bis 3, **dadurch gekennzeichnet, dass** sie mindestens einen (C1 - C10)-Alkylester einer mehrwertigen Carbonsäure, bevorzugt einer mehrwertigen Hydroxycarbonsäure, insbesondere Triethylcitrat, enthält.

5. Zusammensetzung gemäß einem oder mehreren der Ansprüche 1 bis 4, **dadurch gekennzeichnet, dass** sie als Lösungsmittel Ethanol, Ethylacetat, Propanol, Isopropanol, 2-Phenoxy-ethanol, Aceton, Butylacetat und Mischungen davon sowie ggf. bis zu 15 Gew.-% Wasser (bezogen auf das Gesamtgewicht der Lösungsmittel) enthält.

6. Zusammensetzung gemäß einem oder mehreren der Ansprüche 1 bis 5, **dadurch gekennzeichnet, dass** sie mindestens einen pharmazeutischen oder kosmetischen Wirkstoff, vorzugsweise ein Antiseptikum, insbesondere Chlorhexidin, Chlorhexidindigluconat, Chlorhexidindiacetat, Benzalkoniumchlorid, Polyvinylpryrrolidon-lod (PVP-Iod), Polyhexanid und/oder Octenidindihydrochlorid, enthält.

7. Zusammensetzung gemäß einem oder mehreren der Ansprüche 1 bis 6, **dadurch gekennzeichnet, dass** sie Pigmente und/oder Farbstoffe enthält.

8. Zusammensetzung gemäß einem oder mehreren der Ansprüche 1 bis 7, **dadurch gekennzeichnet, das** sie eine Viskosität im Bereich von 1000 bis 40000 mPa·s, bevorzugt von 5000 bis 15000 mPa·s, aufweist.

9. Zusammensetzung gemäß einem oder mehreren der Ansprüche 1 bis 8, **dadurch gekennzeichnet, dass** sie auf der Haut Filme mit einer Wasserdampfdurchlässigkeit von 400 bis 1350 g/m²/24 h, bestimmt nach EN 13726-2, bildet.

10. Vorrichtung zur Verpackung und zum Aufbringen einer flüssigen Zusammensetzung gemäß einem oder mehreren der Ansprüche 1 bis 9, welche einen die Zusammensetzung enthaltenden Behälter aufweist und welche mit einer Auftragskonstruktion versehen ist, **dadurch gekennzeichnet, dass** es sich um einen Stift (Pen) handelt, der einen mechanisch betätigten Kolben und eine elastische, mit mindestens einer Austrittsöffnung, vorzugsweise mit mehreren Austrittsöffnungen, versehene Spitze aufweist.

11. Vorrichtung gemäß Anspruch 10, **dadurch gekennzeichnet, dass** sie einen Deckel aufweist, der die Auftragskonstruktion verschließen kann.

12. Verfahren zum Auftragen einer Zusammensetzung gemäß einem oder mehreren der Ansprüche 1 bis 9, bei welchem mittels einer Vorrichtung gemäß Anspruch 10 oder 11 auf die Haut aufgetragen wird.

13. Verwendung einer Vorrichtung gemäß Anspruch 10 oder 11 zur Verpackung und zum Auftragen einer Zusammensetzung gemäß einem oder mehreren der Ansprüche 1 bis 9.
